# FASCICULE DE BREVET EUROPEEN

(11) **EP 3 316 933 B1**
(45) Date de publication et mention de la délivrance du brevet: **07.08.2019**
(21) Numéro de dépôt: 16742362.3
(22) Date de dépôt: 30.06.2016
(51) Int. Cl.: F16L 17/04, A61M 5/20, A61M 5/31, A61M 5/30

(54) **DISPOSITIF D'INJECTION SANS AIGUILLE À JOINT D'ÉTANCHÉITÉ PERFECTIONNÉ**
NADELLOSER INJEKTOR MIT VERBESSERTER DICHTUNG
NEEDLELESS INJECTOR WITH IMPROVED SEAL

(30) Priorité: 30.06.2015 FR 1556161
(43) Date de publication de la demande: 09.05.2018
(73) Titulaire: Crossject, 21000 Dijon (FR)
(72) Inventeur: VIVIEN, Gilles, 92240 Malakof (FR); VIGOT, Xavier, 21260 Veronnes (FR)
(74) Mandataire: Chevalier, Renaud Philippe
(86) Numéro de dépôt international: PCT/FR2016/051658
(87) Numéro de publication internationale: WO 2017/001797

(56) Documents cités:
- FR-A1- 2 745 619
- FR-A1- 2 815 544
- FR-A1- 2 853 837
- FR-A1- 2 941 027
- US-A- 5 833 243

## Description

L'invention concerne un dispositif d'injection sans aiguille comportant une buse d'injection équipée d'un joint d'étanchéité perfectionné.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans le corps du dispositif et qui est obturé par un piston amont et un piston aval entre lesquels est contenu le principe actif liquide.

L'extrémité libre aval du réservoir coopère avec une buse d'injection qui délimite au moins un canal d'injection s'étendant axialement suivant un axe d'injection.

La buse d'injection est délimitée axialement par une face supérieure en appui axial sur le réservoir, et une face inférieure adaptée pour coopérer avec un opercule de fermeture.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appui la buse d'injection sur sa peau.

Le déplacement du corps dans le capot permet le déclenchement du générateur de gaz, générant un gaz sous pression qui entraîne en déplacement les pistons pour injecter le principe actif à travers la peau du patient, en passant par la buse d'injection.

Pour assurer l'étanchéité entre le réservoir et la buse, notamment au moment de l'injection, il est connu d'utiliser un joint supérieur d'étanchéité.

Le joint supérieur d'étanchéité s'étend autour des canaux d'injection et il est logé dans une gorge supérieure ménagée sur la face supérieure de la buse, ce joint étant conçu pour être comprimé axialement entre la face inférieure du réservoir et la face supérieure de la buse.

De façon connue, le joint présente une forme bombée convexe qui est écrasée axialement par le réservoir.

On constate que lorsque le joint est comprimé entre la buse et le tube, le joint peut déborder de sa gorge et fluer vers l'extérieur entre la buse et le réservoir, créant ainsi un jeu favorable à la fuite de liquide.

De plus, ce type de joint forme généralement des lobes qui contournent les canaux d'injection de la buse, donnant une forme sinueuse au joint, autour de l'axe d'injection.

Une telle forme sinueuse peut favoriser l'apparition de pincements du joint lorsqu'il est comprimé par le réservoir, pouvant provoquer des fuites entre la buse et le réservoir.

La présente invention vise notamment à résoudre ces inconvénients et se rapporte pour ce faire à un dispositif d'injection sans aiguille comportant un système d'injection qui s'étend axialement suivant un axe d'injection et qui comprend au moins, d'amont en aval suivant le sens d'injection :
- au moins un piston,
- un réservoir de principe actif qui présente une face inférieure,
- une buse d'injection qui est délimitée axialement par une face supérieure agencée en regard de la face inférieure du réservoir, et une face inférieure adaptée pour coopérer avec un opercule, la buse délimitant un canal d'injection s'étendant globalement axialement depuis la face supérieure, jusqu'à la face inférieure de la buse,
- un joint supérieur d'étanchéité qui s'étend autour de l'au moins un canal, et qui est logé dans une gorge supérieure ménagée sur la face supérieure de la buse, ledit joint étant conçu pour être en appui axial sur la face inférieure du réservoir, caractérisé en ce que le joint supérieur présente une section radiale en forme de vague qui comprend successivement, suivant une direction radiale, une portion en creux et une portion en bosse, ladite portion en bosse étant adaptée pour être couchée vers la portion en creux prévue à cet effet, sous l'effet de la pression axiale exercée par la face inférieure du réservoir sur ledit joint d'étanchéité.

Le joint supérieur selon l'invention permet d'améliorer l'étanchéité de la buse, en se couchant et non en s'écrasant, ce qui permet notamment d'éviter le pincement et le fluage du joint vers l'extérieur.

Selon une autre caractéristique, la portion en bosse présente un flanc oblique qui est agencé à l'opposé de la portion en creux et qui forme une rampe périphérique adaptée pour permettre le couchage de la portion en bosse du joint supérieur vers la portion en creux, sous l'effet de la pression axiale exercée par le réservoir sur ladite rampe du joint supérieur.

La rampe favorise le couchage du joint supérieur, en coopérant avec la face inférieure du réservoir.

Avantageusement, la portion en creux du joint supérieur est agencée radialement vers l'intérieur de la buse, et la portion en bosse du joint supérieur est agencée radialement vers l'extérieur de la buse.

Cette caractéristique permet au joint supérieur de se coucher vers le centre de la buse.

De plus, la portion en creux présente une forme globalement complémentaire à la forme de la portion en bosse dudit joint supérieur.

Selon une autre caractéristique, le canal d'injection est agencé de façon désaxée par rapport à l'axe d'injection, et le joint supérieur d'étanchéité forme au moins un lobe qui contourne ledit canal d'injection.

Bien que le joint supérieur présente une forme tortueuse, il assure l'étanchéité entre la buse et le récipient en se couchant.

Selon un exemple de réalisation, la buse d'injection délimite trois canaux d'injection qui sont répartis angulairement de façon régulière autour de l'axe d'injection, et en ce que le joint supérieur forme trois lobes qui contournent chacun un desdits canaux, de sorte que le joint supérieur présente une forme globalement circulaire qui présente trois portions de cercle formant chacune un lobe.

Selon un exemple de réalisation préféré, le joint supérieur est réalisé par surmoulage sur la buse.

De plus, le joint supérieur est réalisé en élastomère thermoplastique adapté pour adhérer chimiquement sur la buse réalisée en polycarbonate.

Aussi, le dispositif selon l'invention comporte un joint inférieur supplémentaire qui s'étend sur la face inférieure de la buse, et qui est adapté pour assurer l'étanchéité avec un opercule de fermeture de la buse, le joint inférieur étant réalisé venu de matière avec le joint supérieur, lesdits joints, étant reliés entre eux par au moins un passage, prévu à cet effet, ménagé dans la buse.

Selon un exemple de réalisation, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en perspective éclatée axialement, qui illustre un dispositif d'injection sans aiguille, selon l'invention ;
- la figure 2 est une vue en perspective de dessus qui illustre la buse de la figure 1 équipée d'un joint supérieur d'étanchéité ;
- la figure 3 est une vue schématique en coupe transversale qui illustre la buse de la figure 1 équipée d'un joint d'étanchéité ;
- la figure 4 est une vue en perspective de dessous, qui illustre la face inférieure de la buse d'injection munie d'un joint inférieur ;
- la figure 5 est une vue de détail en section transversale, qui illustre une portion du joint de la figure 2 au repos ;
- la figure 6 est une vue de détail en section transversale, qui illustre une portion du joint de la figure 2 couché par le réservoir ;
- la figure 7 est une vue en perspective qui illustre le joint de la figure 2 dans son intégralité.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe B d'injection du dispositif d'injection.

Sur l'ensemble de ces figures, des références identiques ou analogues représentent des organes ou ensembles d'organes identiques ou analogues.

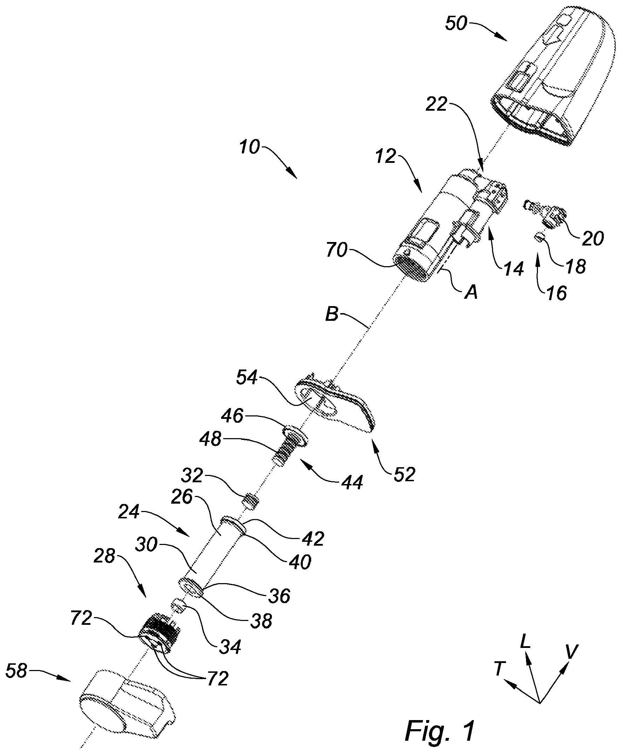

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston amont 32 et un piston aval 34 entre lesquels est contenu le principe actif 26, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

Aussi, selon la figure 1, une membrane 44 souple cylindrique comporte un siège annulaire 46 qui est interposé axialement entre la face supérieure 40 du réservoir 24 et l'orifice de sortie de la chambre d'expansion 22, et un corps 48 cylindrique qui s'étend axialement à l'intérieur du réservoir 24, au dessus du piston amont 32.

Le corps 48 de la membrane 44 est conçu pour s'étendre axialement, sous l'effet de la pression du gaz généré par le générateur de gaz 16, pour pousser le piston amont 32.

En référence à la figure 1, le corps 12 est enveloppé par un capot 50 creux qui délimite une ouverture inférieure fermée par une semelle 52 horizontale formant fond de capot.

La semelle 52 délimite un passage circulaire 54 autour de l'axe B d'injection qui est adapté pour le passage de la buse 28 d'injection et de l'extrémité aval du corps 12, de sorte que la buse 28 comporte un tronçon inférieur faisant saillie verticalement vers le bas hors du capot 50.

Aussi, le dispositif 10 d'injection est équipé d'un bouchon 58 qui est monté sur le corps 12 de façon amovible par un moyen de verrouillage du type à baïonnette.

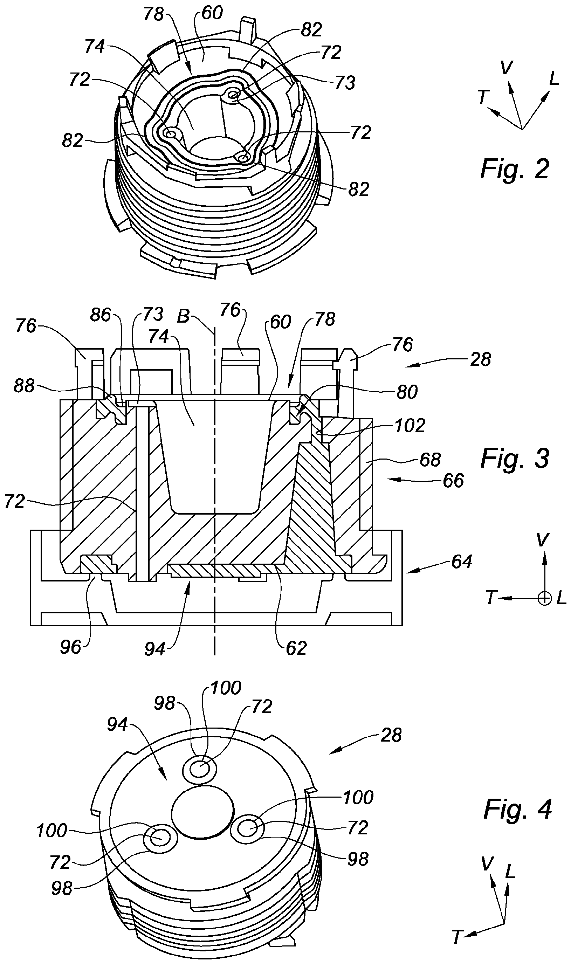

La buse 28 d'injection, illustrée aux figures 2 et 3, présente une forme globalement cylindrique qui s'étend axialement suivant l'axe B d'injection depuis une face supérieure 60 en appui axial sur la face inférieure 38 du réservoir 24, jusqu'à une face inférieure 62 d'injection adaptée pour coopérer avec un opercule 64 de fermeture.

La face périphérique 66 cylindrique de la buse 28 présente un filetage 68 conçu pour visser la buse 28 sur l'extrémité libre du corps 12 équipé d'un taraudage 70 complémentaire visible à la figure 1.

La buse 28 délimite trois canaux 72 d'injection axiaux qui s'étendent parallèlement à l'axe B d'injection et qui sont agencés angulairement de façon régulière autour de l'axe B d'injection, chaque canal 72 débouchant dans la face supérieure 60 et dans la face inférieure 62 de la buse 28.

L'extrémité libre inférieure de chaque canal 72 forme une embouchure saillante axialement depuis la face inférieure 62 de la buse 28.

De plus, la buse 28 délimite un logement 74 central qui est adapté pour recevoir le piston aval 34 à la suite du déclenchement de l'injection.

Plus particulièrement, l'extrémité libre supérieure de chaque canal 72 forme un évasement 73 qui communique avec le logement 74, pour permettre au principe actif 26 de pénétrer dans chaque canal 72, depuis le logement 74.

En effet, lorsque le générateur de gaz 16 est déclenché, le gaz sous pression pousse la colonne de liquide formée par le piston amont 32, le principe actif 26 et le piston aval 34, le piston aval 34 tombant dans le logement 74 de la buse 28 prévu à cet effet pour permettre au principe actif 26 de s'écouler à travers les canaux 72.

Aussi, la buse 28 est équipé de trois crochets 76 qui s'étendent vers le haut depuis la face supérieure 60 de la buse 28 et qui sont adaptés pour coopérer avec la collerette inférieure 36 du réservoir 24.

Pour assurer l'étanchéité entre la buse 28 et le réservoir 24, le dispositif d'injection 10 comporte un joint supérieur 78 d'étanchéité qui s'étend autour de l'axe B d'injection, et autour des trois canaux 72, le joint supérieur 78 étant logé dans une gorge supérieure 80 complémentaire ménagée sur la face supérieure 60 de la buse 28.

Comme on peut le voir à la figure 2, le joint supérieur 78 forme trois lobes 82 qui contournent chacun un canal 72 associé, de sorte que le joint supérieur 78 présente une forme globalement circulaire qui présente trois portions de cercle formant les lobes 82.

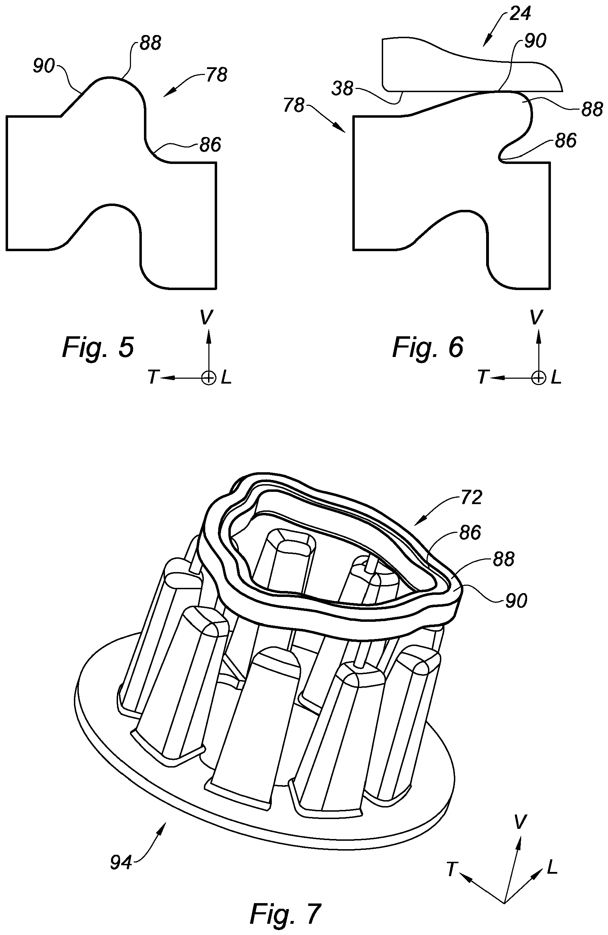

En référence aux figure 3 et 5, le joint supérieur 78 présente une section radiale en forme de vague qui comprend successivement, suivant une direction radiale transversale, une portion en creux 86 agencée radialement vers le centre de la buse 28, et une portion en bosse 88 agencée radialement vers l'extérieur de la buse 28.

La portion en bosse 88 est adaptée pour être couchée vers la portion en creux 86 prévue à cet effet, sous l'effet de la pression axiale exercée par la face inférieure 38 du réservoir 24 sur le joint supérieur 78, comme illustré à la figure 6.

A cet effet, la portion en bosse 88 du joint supérieur 78 présente un flanc oblique qui est agencé à l'opposé de la portion en creux et qui forme une rampe 90 périphérique adaptée pour permettre l'abattement, ou le couchage, de la portion en bosse 88 vers la portion en creux 86, sous l'effet de la pression axiale exercée par le réservoir 24 sur la rampe 90.

La rampe 90 s'étend de bas en haut, radialement vers le centre de la buse 28.

Le terme « couchée », concernant la portion en bosse 88 du joint supérieur 78, signifie ici que la portion en bosse 88 s'incline vers la portion en creux 86, à la différence d'un joint qui s'écrase et flue.

De plus, la portion en creux 86 présente une forme globalement complémentaire à la forme de la portion en bosse 88 du joint supérieur, formant un logement dans lequel la portion en bosse 88 peut se coucher, au moins en partie.

Selon un autre aspect illustré aux figures 4 et 7, le dispositif 10 d'injection comporte un joint inférieur 94 supplémentaire qui s'étend sur la face inférieure 62 de la buse 28 et qui est adapté pour assurer l'étanchéité avec l'opercule 64 de fermeture de la buse 28.

Dans ce but, comme on peut le voir à la figure 3, le joint inférieur 94 présente une forme de disque plan radial qui est adapté pour être en appui axial sur un bourrelet 96 annulaire, saillant axialement, qui est formé par l'opercule 64 et qui s'étend autour de l'axe B d'injection.

Aussi, le joint inférieur 94 délimite trois trous 98 pour le passage des embouchures 100 de chaque canal 72.

Le joint inférieur 94 et le joint supérieur 78 sont réalisés venus de matière par surmoulage sur la buse 28, en élastomère thermoplastique.

A cet effet, la buse 28 délimite trois passages 102 (dont un est représenté figure 3) qui relient chacun la gorge supérieure 80 ménagée sur la face supérieure 60 de la buse 28 et la face inférieure 62 de la buse 28, pour permettre la réalisation en une seule pièce du joint inférieur 94 et du joint supérieur 78 par surmoulage sur la buse 28.

Avantageusement, la buse 28 est réalisée par moulage en polycarbonate et l'élastomère utilisé pour réaliser le joint supérieur 78 et le joint inférieur 94 est adapté pour adhérer chimiquement avec le polycarbonate formant la buse 28.

Selon un exemple de réalisation préféré, le joint supérieur 78 et le joint inférieur 94 sont réalisés en thermoplastique élastomère d'une dureté « Shore A » de 70, et d'un allongement à la rupture de 650 pourcent.

## Revendications

1. Dispositif (10) d'injection sans aiguille comportant un système d'injection (28) qui s'étend axialement suivant un axe (B) d'injection et qui comprend au moins, d'amont en aval suivant le sens d'injection :
- au moins un piston (32, 34),
- un réservoir (24) qui contient un principe actif (26) et qui présente une face inférieure (38),
- une buse (28) d'injection qui est délimitée axialement par une face supérieure (60) agencée en regard de la face inférieure (38) du réservoir (24), et une face inférieure (62) adaptée pour coopérer avec un opercule (64), la buse (28) délimitant au moins un canal (72) d'injection s'étendant globalement axialement depuis la face supérieure (60), jusqu'à la face inférieure (62) de la buse (28),
- un joint supérieur (78) d'étanchéité qui s'étend autour de l'au moins un canal (72), et qui est logé dans une gorge supérieure (80) ménagée sur la face supérieure (60) de la buse (28), ledit joint (78) étant conçu pour être en appui axial sur la face inférieure (38) du réservoir (24),
**caractérisé en ce que** le joint supérieur (78) présente une section radiale en forme de vague qui comprend successivement, suivant une direction radiale, une portion en creux (86) et une portion en bosse (88), ladite portion en bosse (88) étant adaptée pour être couchée vers la portion en creux (86) prévue à cet effet, sous l'effet de la pression axiale exercée par la face inférieure (38) du réservoir (24) sur ledit joint (78) d'étanchéité.

2. Dispositif (10) d'injection sans aiguille selon la revendication 1, **caractérisé en ce que** la portion en bosse (88) présente un flanc oblique qui est agencé à l'opposé de la portion en creux (86) et qui forme une rampe (90) périphérique adaptée pour permettre le couchage de la portion en bosse (88) du joint supérieur (78) vers la portion en creux (86), sous l'effet de la pression axiale exercée par le réservoir (24) sur ladite rampe (90) du joint supérieur (78).

3. Dispositif (10) d'injection sans aiguille selon l'une quelconque des revendications précédentes, **caractérisé en ce que** la portion en creux (86) du joint supérieur (78) est agencée radialement vers l'intérieur de la buse (28), et la portion en bosse (88) du joint supérieur (78) est agencée radialement vers l'extérieur de la buse (28).

4. Dispositif (10) d'injection sans aiguille selon l'une quelconque des revendications précédentes, **caractérisé en ce que** la portion en creux (86) présente une forme globalement complémentaire à la forme de la portion en bosse (88) dudit joint supérieur (78).

5. Dispositif (10) d'injection sans aiguille selon l'une quelconque des revendications précédentes, **caractérisé en ce que** le canal (72) d'injection est agencé de façon désaxée par rapport à l'axe (B) d'injection, et **en ce que** le joint supérieur (78) d'étanchéité forme au moins un lobe (82) qui contourne ledit canal (72) d'injection.

6. Dispositif (10) d'injection sans aiguille selon la revendication 5, **caractérisé en ce que** la buse (28) d'injection délimite trois canaux (72) d'injection qui sont répartis angulairement de façon régulière autour de l'axe (B) d'injection, et **en ce que** le joint supérieur (78) forme trois lobes (82) qui contournent chacun un desdits (72) canaux, de sorte que le joint supérieur (78) présente une forme globalement circulaire qui présente trois portions de cercle formant chacune un lobe (82).

7. Dispositif (10) d'injection sans aiguille selon l'une quelconque des revendications précédentes, **caractérisé en ce que** le joint supérieur (78) est réalisé par surmoulage sur la buse (28).

8. Dispositif (10) d'injection sans aiguille selon l'une quelconque des revendications précédentes, **caractérisé en ce que** le joint supérieur (78) est réalisé en élastomère thermoplastique adapté pour adhérer chimiquement sur la buse (28) réalisée en polycarbonate.

9. Dispositif (10) d'injection sans aiguille selon l'une quelconque des revendications précédentes, **caractérisé en ce qu'**il comporte un joint inférieur (94) supplémentaire qui s'étend sur la face inférieure (62) de la buse (28), et qui est adapté pour assurer l'étanchéité avec un opercule (64) de fermeture de la buse (28), le joint inférieur (94) étant réalisé venu de matière avec le joint supérieur (78), lesdits joints (78, 94) étant reliés entre eux par au moins un passage (102), prévu à cet effet, ménagé dans la buse (28).

10. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadellose Injektionsvorrichtung (10), die ein Injektionssystem (28) umfasst, welches sich axial entlang einer Injektionsachse (B) erstreckt, und die in der Injektionsrichtung von stromaufwärts nach stromabwärts mindestens umfasst:
- mindestens einen Kolben (32, 34),
- einen Behälter (24), der einen Wirkstoff (26) enthält und der eine untere Seite (38) aufweist,
- eine Injektionsdüse (28), die axial von einer der unteren Seite (38) des Behälters (24) zugewandt angeordneten oberen Seite (60) und einer unteren Seite (62) begrenzt wird, die dafür geeignet ist, mit einem Deckel (64) zusammenzuwirken, wobei die Düse (28) mindestens einen Injektionskanal (72) begrenzt, der sich allgemein axial von der oberen Seite (60) bis zur unteren Seite (62) der Düse (28) erstreckt,
- eine obere Dichtung (78), die sich um den mindestens einen Kanal (72) herum erstreckt und die in einer oberen Kehle (80) aufgenommen ist, welche an der oberen Seite (60) der Düse (28) gestaltet ist, wobei die Dichtung (78) dafür konzipiert ist, axial an der unteren Seite (38) des Behälters (24) anzuliegen,
**dadurch gekennzeichnet, dass** die obere Dichtung (78) einen radialen Querschnitt in Wellenform aufweist, der nacheinander in einer radialen Richtung einen hohlen Abschnitt (86) und einen erhabenen Abschnitt (88) aufweist, wobei der erhabene Abschnitt (88) dafür geeignet ist, unter der Wirkung des axialen Drucks, der von der unteren Seite (38) des Behälters (24) auf die Dichtung (78) ausgeübt wird, zu dem hohlen Abschnitt (86) hin umgelegt zu werden, der zu diesem Zweck vorgesehen ist.

2. Nadellose Injektionsvorrichtung (10) nach Anspruch 1, **dadurch gekennzeichnet, dass** der erhabene Abschnitt (88) eine schräge Flanke aufweist, die dem hohlen Abschnitt (86) gegenüber angeordnet ist und die eine umlaufende Rampe (90) bildet, die dafür geeignet ist, das Umlegen des erhabenen Abschnitts (88) der oberen Dichtung (78) unter der Wirkung des axialen Drucks, der vom Behälter (24) auf die Rampe (90) der oberen Dichtung (78) ausgeübt wird, zum hohlen Abschnitt (86) hin zu ermöglichen.

3. Nadellose Injektionsvorrichtung (10) nach einem der vorstehenden Ansprüche, **dadurch gekennzeichnet, dass** der hohle Abschnitt (86) der oberen Dichtung (78) radial zur Innenseite der Düse (28) hin angeordnet ist, und der erhabene Abschnitt (88) der oberen Dichtung (78) radial zur Außenseite der Düse (28) hin angeordnet ist.

4. Nadellose Injektionsvorrichtung (10) nach einem der vorstehenden Ansprüche, **dadurch gekennzeichnet, dass** der hohle Abschnitt (86) eine Form aufweist, die allgemein zur Form des erhabenen Abschnitts (88) der oberen Dichtung (78) komplementär ist.

5. Nadellose Injektionsvorrichtung (10) nach einem der vorstehenden Ansprüche, **dadurch gekennzeichnet, dass** der Injektionskanal (72) in Bezug auf die Injektionsachse (B) außeraxial angeordnet ist, und dadurch, dass die obere Dichtung (78) mindestens eine Nase (82) bildet, die um den Injektionskanal (72) herumläuft.

6. Nadellose Injektionsvorrichtung (10) nach Anspruch 5, **dadurch gekennzeichnet, dass** die Injektionsdüse (28) drei Injektionskanäle (72) begrenzt, die winkelmäßig gleichmäßig um die Injektionsachse (B) herum verteilt sind, und dadurch, dass die obere Dichtung (78) drei Nasen (82) bildet, die jede um einen der Kanäle (72) herumlaufen, sodass die obere Dichtung (78) eine allgemein runde Form aufweist, die drei Kreisabschnitte aufweist, welche jeder eine Nase (82) bilden.

7. Nadellose Injektionsvorrichtung (10) nach einem der vorstehenden Ansprüche, **dadurch gekennzeichnet, dass** die obere Dichtung (78) durch Aufformen an der Düse (28) ausgeführt ist.

8. Nadellose Injektionsvorrichtung (10) nach einem der vorstehenden Ansprüche, **dadurch gekennzeichnet, dass** die obere Dichtung (78) aus thermoplastischem Elastomer ausgeführt ist, das dafür geeignet ist, chemisch an der aus Polycarbonat ausgeführten Düse (28) zu haften.

9. Nadellose Injektionsvorrichtung (10) nach einem der vorstehenden Ansprüche, **dadurch gekennzeichnet, dass** sie eine zusätzliche untere Dichtung (94) umfasst, die sich an der unteren Seite (62) der Düse (28) erstreckt und die dafür geeignet ist, die Dichtigkeit mit einem Verschlussdeckel (64) der Düse (28) sicherzustellen, wobei die untere Dichtung (94) einstückig mit der oberen Dichtung (78) hergestellt ist, wobei die Dichtungen (78, 94) durch mindestens einen zu diesem Zweck vorgesehenen Durchgang (102), der in der Düse (28) gestaltet ist, miteinander verbunden sind.

10. Nadellose Injektionsvorrichtung (10) nach einem der vorstehenden Ansprüche, **dadurch gekennzeichnet, dass** der im Behälter (24) enthaltene Wirkstoff (26) ausgewählt ist aus der Gruppe, die die folgenden Wirkstoffe umfasst:
- Methotrexat,
- Adrenalin,
- Sumatriptan,
- Hydrocortison,
- Naloxon,
- Midazolam,
- Apomorphin,
- Ethylnatrexon-Bromid,
- Phytomenadion,
- Chlorpromazin-Hydrochlorid,
- Zuclopenthixol-Acetat,
- Danaparoid-Natrium,
- Enoxaparin-Natrium,
- Estradiol-Cipionat,
- Medoxyprogesteron-Acetat,
- Medroparin-Calcium,
- Methylprednisolon-Acetat
- Heparin-Calcium,
- Terbulin.

## Claims

1. A needleless injection device (10) including an injection system (28) which extends axially along an injection axis (B) and which comprises at least, from upstream to downstream in the direction of injection:
- at least one plunger (32, 34),
- a reservoir (24) which contains an active ingredient (26) and which has a lower face (38),
- an injection nozzle (28) which is axially delimited by an upper face (60) arranged opposite the lower face (38) of the reservoir (24), and a lower face (62) adapted to cooperate with a lid (64), the nozzle (28) delimiting at least one injection channel (72) extending generally axially from the upper face (60) to the lower face (62) of the nozzle (28),
- an upper sealing gasket (78) which extends around the at least one channel (72) and which is housed within an upper groove (80) formed on the upper face (60) of the nozzle (28); said gasket (78) being designed to axially bear on the lower face (38) of the reservoir (24),
**characterized in that** the upper gasket (78) has a wave-like shaped radial section which successively comprises, in a radial direction, a recessed portion (86) and a humped portion (88), said humped portion (88) being adapted to be laid towards the recessed portion (86) provided for this purpose, under the effect of the axial pressure exerted by the lower face (38) of the reservoir (24) on said sealing gasket (78).

2. The needleless injection device (10) according to claim 1, **characterized in that** the humped portion (88) has an oblique flank which is arranged opposite to the recessed portion (86) and which forms a peripheral ramp (90) adapted to enable the humped portion (88) of the upper gasket (78) to be laid towards the recessed portion (86) under the effect of the axial pressure exerted by the reservoir (24) on said ramp (90) of the upper gasket (78).

3. The needleless injection device (10) according to any one of the preceding claims, **characterized in that** the recessed portion (86) of the upper gasket (78) is arranged radially inwards the nozzle (28) and the humped portion (88) of the upper gasket (78) is arranged radially outwards the nozzle (28).

4. The needleless injection device (10) according to any one of the preceding claims, **characterized in that** the recessed portion (86) has a shape generally complementary to the shape of the humped portion (88) of said upper gasket (78).

5. The needleless injection device (10) according to any one of the preceding claims, **characterized in that** the injection channel (72) is arranged in an offset manner relative to the injection axis (B), and **in that** the upper sealing gasket (78) forms at least one lobe (82) which bypasses said injection channel (72).

6. The needleless injection device (10) according to claim 5, **characterized in that** the injection nozzle (28) delimits three injection channels (72) which are distributed angularly in a regular manner around the injection axis (B), and **in that** the upper gasket (78) forms three lobes (82) each bypassing one of said channels (72), so that the upper gasket (78) has a generally circular shape which has three circle portions each forming a lobe (82).

7. The needleless injection device (10) according to any one of the preceding claims, **characterized in that** the upper gasket (78) is made by overmolding on the nozzle (28).

8. The needleless injection device (10) according to any one of the preceding claims, **characterized in that** the upper gasket (78) is made of a thermoplastic elastomer adapted to adhere chemically to the nozzle (28) made of polycarbonate.

9. The needleless injection device (10) according to any one of the preceding claims, **characterized in that** it includes an additional lower gasket (94) which extends on the lower face (62) of the nozzle (28), and which is adapted to ensure sealing with a closure lid (64) of the nozzle (28), the lower gasket (94) being formed integrally with the upper gasket (78), said gaskets (78, 94) being interconnected by at least one passage (102) provided for this purpose, formed in the nozzle (28).

10. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate
- Heparin calcium,
- Terbulin.
